# EUROPEAN PATENT APPLICATION

(11) **EP 4 488 373 A1**
(43) Date of publication of application: **08.01.2025**
(21) Application number: 23183405.2
(22) Date of filing: 04.07.2023
(51) Int. Cl.: C12N 15/62, C07K 14/47, C12N 9/10, C12N 9/22

(54) **SCAN-BASED FUSION PROTEINS FOR MODULATING GENE EXPRESSION AND METHODS OF USE**

(71) Applicant: Ecole Polytechnique Fédérale de Lausanne (EPFL), 1015 Lausanne (CH)
(72) Inventor: TRONO, Didier, 1134 Vufflens-Chateau (CH); BEGNIS, Martina, 1260 Nyon (CH)
(74) Representative: KATZAROV S.A.

(57) **Abstract**

The invention provides fusion protein comprising a SCAN domain bearing member of a zinc finger-containing protein, a fragment or variant thereof and a catalytically inactive RNA-guided endonuclease or a DNA adenine methyltransferase. Preferably, the fusion protein is for use in the treatment and/or prevention of cancer, neurodegenerative disease, neurodevelopmental disease, aging and aging-related disease.

## Description

### FIELD OF THE INVENTION

The invention provides fusion protein comprising a SCAN domain bearing member of a zinc finger-containing protein (ZFP), a fragment or variant thereof and a catalytically inactive RNA-guided endonuclease or a DNA adenine methyltransferase. Preferably, the fusion protein is for use in the treatment and/or prevention of cancer, neurodegenerative disease, neurodevelopmental disease, aging and aging-related disease.

### BACKGROUND OF THE INVENTION

The eukaryotic genome is spatially organized to guarantee the orderly execution of transcriptional programs. The genome is dynamically distributed between euchromatin and heterochromatin, with hundreds of genes switching between these respectively active and inactive compartments during development and differentiation. Nuclear bodies can serve as a genomic scaffold for this reorganization by sequestering chromatin-regulating proteins. In the vast majority of nuclei, heterochromatin marked by H3K27me3 or H3K9me3 gathers at the nuclear lamina (NL) or around the nucleolus via an actively regulated dynamic process. While lamina-associated domains (LADs) have been extensively mapped, nucleolus-associated domains (NADs) are less characterized.

Previous studies revealed that, in addition to centromeric and peri centromeric regions, NADs are enriched in specific subsets of human genes, including those coding for zinc finger proteins, protocadherins, immunoglobulins and olfactory receptors (OR), which are organised in large clusters and expressed in a tissue-specific manner. In mice, the association of developmentally regulated genes with NADs varies between different cell types. These observations led to the hypothesis that the nucleolus acts as a specialized compartment to establish repressive chromatin and controls the dynamic execution of lineage-specific expression programs. However, the molecular forces behind NADs organization have so far remained elusive.

There exists a need for alternative methods and compounds to edit the spatial organization of target genomic loci, particularly with regards to membraneless nuclear compartments such as nucleoli.

### SUMMARY OF THE INVENTION

The present invention provides fusion protein comprising i) a SCAN domain bearing member of a zinc finger-containing protein, a fragment or variant thereof and ii) a catalytically inactive RNA-guided endonuclease or iii) a DNA adenine methyltransferase.

Further provided is an isolated polynucleotide encoding a fusion protein of the invention, a plasmid or a vector comprising said nucleic acid sequence, and a prokaryotic or eukaryotic host cell, or population of cells, comprising the plasmid or vector.

Further provided is a pharmaceutical composition comprising a therapeutically effective amount of at least one fusion protein and at least one or more gRNAs that bind the catalytically inactive endonuclease, a polynucleotide, a plasmid or vector, or a prokaryotic or eukaryotic host cell, or population of cells, and a pharmaceutically acceptable carrier, diluent and/or excipient.

Further provided are methods of treatment and/or prevention of a disease comprising administering to a subject in need thereof, a i) pharmaceutical composition of the invention, ii) a fusion protein along with at least one or more gRNAs of the invention, iii) a polynucleotide of the invention, iv) a plasmid or vector of the invention, or v) a prokaryotic or eukaryotic host cell, or population of cells.

### DESCRIPTION OF THE FIGURES

Figure 1: Schematic representation of dCas9-KRAB-SCAN fusion protein.
Figure 2: Schematic representation of dCas9-SCAN fusion protein.
Figure 3: Schematic representation of DamID-SCAN fusion protein.
Figure 4: CRISPR-S silencing of POU5F1B gene in LS1034 cell line, PCR primers P1
Figure 5: CRISPR-S silencing of POU5F1B gene in LS1034 cell line, PCR primers P2.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise(s)" and "comprising" also encompass the more restricted ones "consist(s)", "consisting" as well as "consist/consisting essentially of", respectively.

As used in the specification and claims, the singular form "a", "an" and "the" include plural references unless the context clearly dictates otherwise.

As used herein, "at least one" or "at least one or more" means "one or more", "two or more", "three or more", etc. For example, at least one or more gRNAs means one sgRNA, two sgRNAs, three sgRNAs, or more, etc...

As used herein the terms "subject"/"subject in need thereof", or "patient"/"patient in need thereof" are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human suffering from a cancer, a neurodegenerative disease, a neurodevelopmental disease, aging and aging-related disease or a human that might be at risk of suffering from a cancer, a neurodegenerative disease, a neurodevelopmental disease, aging and aging-related disease.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity, i.e., an analogue of A will base-pair with T.

The term "vector", as used herein, refers to a viral vector or to a nucleic acid (DNA or RNA) molecule such as a plasmid or any other vehicle, which contains one or more heterologous nucleic acid sequences of the invention and, preferably, is designed for transfer between different host cells. The terms "expression vector", "gene delivery vector" and "gene therapy vector" refer to any vector that is effective to incorporate and express one or more nucleic acid(s) of the invention, in a cell, preferably under the regulation of a promoter. A cloning or expression vector may comprise additional elements, for example, regulatory and/or post-transcriptional regulatory elements in addition to a promoter.

The term "about," particularly in reference to a given quantity, number or percentage, is meant to encompass deviations of plus or minus ten percent (± 10). For example, about 5% encompasses any value between 4.5% to 5.5%, such as 4.5, 4.6, 4.7, 4.8, 4.9, 5, 4.1, 5.2, 5.3, 5.4, or 5.5.

The terms "KRAB-containing zinc finger proteins", "KZFP", "KRAB-ZnF", and "KRAB-ZFP" are used interchangeably herein to refer to KRAB-containing zinc finger proteins that constitute a large family of transcription factors notably involved in controlling the expression of transposable element-embedded regulator sequences. KZFPs and their genomic targets contribute to regulating many biological events, from early embryogenesis to brain development, and from immune responses to liver metabolism.

While focusing on these KZFP, the Inventors surprisingly discovered the role of the SCAN domain of ZNF274, that is the first identified domain to drive both nucleolar localization and protein dimerization of a transcription factor to regulate the spatial positioning of target genes.

Based on these properties, the Inventors designed SCAN-fusion proteins as well as methods to generate modifications at targeted genomic loci, which include transcriptional repression, DNA proximal tethering and nucleolar repositioning, and to map spatiotemporal genome organization at the nucleolus.

The present invention thus provides one or more fusion protein(s) comprising i) a SCAN domain bearing member of a zinc finger-containing protein, a fragment or variant thereof and ii) a catalytically inactive RNA-guided endonuclease or iii) a DNA adenine methyltransferase.

The term "fusion protein" refers to a chimeric protein created through the covalent in tandem joining of two or more genes, directly or indirectly, that originally coded for separate proteins.

As used herein, the term "SCAN domain" refers to a highly conserved vertebrate-specific motif and it is most often found near the N-terminus of C2H2 zinc finger proteins (Emerson RO, Thomas JH. Gypsy and the birth of the SCAN domain. J Virol. 2011 Nov;85(22):12043-52). SCAN is thought to act as a protein interaction domain mediating self-association or hetero-association with other proteins.

The human genome encodes over 70 SCAN zinc finger proteins (Sander, T.L., Stringer, K.F., Maki, J.L., Szauter, P., Stone, J.R., and Collins, T. (2003). The SCAN domain defines a large family of zinc finger transcription factors. Gene 310, 29-38), and this protein family may have more generally evolved to modulate spatial genome architecture and shape cell-specific regulatory networks. Usually, the SCAN domain is able to drive protein dimerization of a transcription factor.

In one aspect, the SCAN domain is also able to drive nucleolar localization.

In one aspect, the SCAN domain is selected from the non-limiting group comprising the
- SCAN domain of ZNF274 (**SEQ ID NO: 1**),
- ZNF274 comprising the SCAN domain (**SEQ ID NO: 24**),
- the SCAN domain of any other SCAN-containing protein that is able to interact with ZNF274 (for example ZNF24 and SCAND1) or whose perinucleolar tethering depends on ZNF274,
a fragment thereof, a variant thereof, or a combination of one or more thereof.

As used herein, a "fragment" of one or more nucleic acid sequence or polypeptide sequence of the invention refers to a sequence containing less nucleotides or amino acids in length than the respective sequences of the invention while retaining the biological activity or property described herein. Preferably, this fragment contains, at least 10%, 20%, 30%, 40%, 50%, 60%, 70%, 80%, or 90% of the entire length of the reference nucleic acid sequence or polypeptide sequence.

As used herein, the term "variant" refers to biologically active derivatives of a nucleic acid or polypeptide sequence of the invention. In general, the term "variant" refers to molecules having a native sequence and structure with one or more additions, substitutions (generally conservative in nature) and/or deletions (e.g. alternative splicing variants), relative to the native molecule, so long as the modifications do not destroy biological activity described herein and which are "substantially homologous" to the reference molecule. In general, the sequences of such variants are functionally, i.e. biologically, active variants and will have a high degree of sequence homology to the reference sequence, e.g., sequence homology of more than 50%, generally more than 60%-70%, even more particularly 80% or more, such as at least 90% or 95% or more, when the two sequences are aligned. More preferably, an active variant will have a degree of sequence homology to the reference sequence of at least 80%, 81%, 82%, 83%, 84%, 85%, 86%, 87%, 88%, 89%, 90%, 91%, 92%, 93%, 94%, 95%, 96%, 97%, 98%, or 99%.

Non-limiting examples of conservative amino acid substitutions are herein defined as exchanges within one of the following five groups:
I. Small aliphatic, nonpolar or slightly polar residues: Ala, Ser, Thr, Pro, Gly
II. Polar, positively charged residues: His, Arg, Lys
III. Polar, negatively charged residues: and their amides: Asp, Asn, Glu, Gin
IV. Large, aromatic residues: Phe, Tyr, Tip
V. Large, aliphatic, nonpolar residues: Met, Leu, Ile, Val, Cys.

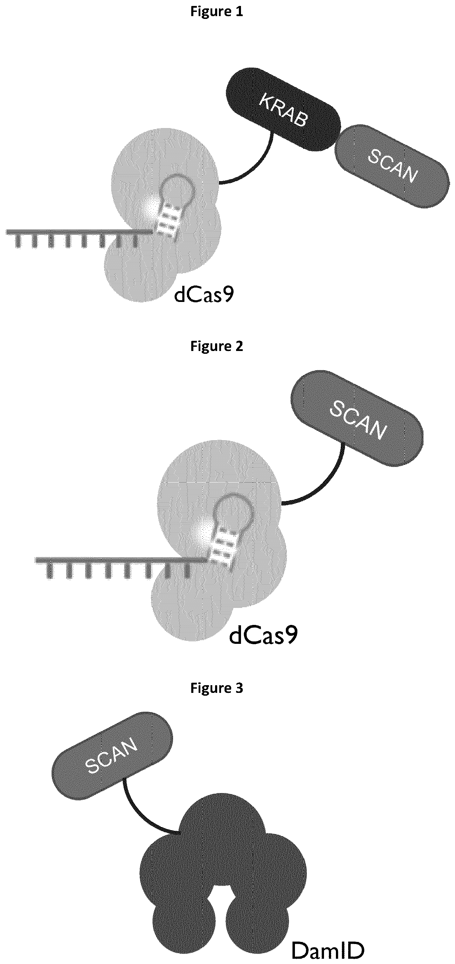

Examples of variants of the SCAN domain can be found, e.g., in Williams AJ, et al., 1999 (see the consensus sequence depicted in Fig. 1 of Williams AJ, Blacklow SC, Collins T. The zinc finger-associated SCAN box is a conserved oligomerization domain. Mol Cell Biol. 1999 Dec;19(12):8526-35), which is incorporated herein by reference in its entirety.

As used herein, "Homology" refers to the percent identity between two polynucleotide or two polypeptide sequences of the invention. Two nucleic acid, or two polypeptide sequences are "substantially homologous" to each other when the sequences exhibit at least about 50% sequence identity, preferably at least about 75% sequence identity, more preferably at least about 80% sequence identity, more preferably at least about 90% sequence identity, and most preferably at least about 95%-98% sequence identity over a defined length of the molecules. As used herein, substantially homologous also refers to sequences showing complete identity to the specified sequence.

In general, "identity" refers herein to an exact nucleotide to nucleotide or amino acid to amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. Sequence identity is typically measured using sequence analysis software (for example, Sequence Analysis Software Package of the Genetics Computer Group, University of Wisconsin Biotechnology Center, 1710 University Avenue, Madison, Wis.53705, BLAST, BESTFIT, GAP, or PILEUP/PRETTYBOX programs). Such software matches identical or similar sequences by assigning degrees of homology to various substitutions, deletions, and/or other modifications.

Alternatively, homology can be determined by readily available computer programs or by hybridization of polynucleotides under conditions which form stable duplexes between homologous regions, followed by digestion with single stranded specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art.

The term "nuclease," as used herein, refers to an agent, for example, a protein, capable of cleaving a phosphodiester bond connecting two nucleotide residues in a nucleic acid molecule. A nuclease may be an endonuclease, cleaving a phosphodiester bond within a polynucleotide chain. Some nucleases, such as endonucleases, are site-specific nucleases, binding and/or cleaving a specific phosphodiester bond within a specific nucleotide sequence.

In one aspect, the endonuclease is a CRISPR associated protein (Cas) endonuclease.

The endonuclease described herein, e.g. a Cas endonuclease, is a catalytically inactive endonuclease (e.g., lacks endonuclease activity) that retains the ability to bind to a specific nucleotide sequence, i.e. a nuclease target site on said nucleotide sequence.

Non-limiting examples of a catalytically inactive Cas endonuclease comprise, but are not limited to, Cas1, Cas3, Cas4, Cas7, Cas9, or Cas10, a fragment, a variant thereof or a combination of one or more thereof.

In some aspects, the catalytically inactive endonuclease is a catalytically inactive Cas9 "CRISPR/dCas9" or "dCas9". The terms "CRISPR/dCas9," "dCas9," "dCas9 nuclease," or "dCas9 endonuclease" are used interchangeably to refer to an RNA-guided catalytically inactive endonuclease comprising a dCas9 protein ("dead Cas9"), or a fragment thereof (e.g., a protein comprising an inactive DNA cleavage domain of Cas9). Preferably, the Cas9 protein comprises at least one domain selected from the group comprising a Rec1 domain, a bridge helix domain, and/or a protospacer adjacent motif (PAM) interacting domain.

In one aspect, the dCas9 protein comprises, or essentially consists of, an amino acid sequence as set forth in **SEQ ID NO: 3**, a fragment or a variant thereof.

In one aspect, the Cas9 is a dCas9 protein comprising a D10A mutation in a RuvC1 domain and/or a H840A mutation in a HNH domain.

In one aspect, the fusion protein further comprises one or more linkers. As used herein, the term "linker" refers to a polypeptide that serves to connect the dCas9 protein with the two or more repressor domains and/or other protein sequences/domains including fluorescent markers, tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. The length of a linker peptide can vary; for example, the length may be as few as one amino acid or more than one hundred amino acids. Non-limiting examples of linker peptides contemplated herein can include tags, self-cleaving, and flexible linkers, such as Gly-Ser linkers, and other similar linkers.

Non-limiting examples of linkers comprise:
- a 3x Flag-tag amino acid sequence, comprising, or consisting essentially of, an amino acid sequence as set forth in **SEQ ID NO: 7** (DYKDHDGDYKDHDIDYKDDDDK), a fragment or a variant thereof,
- a V5 epitope-tag amino acid sequence comprising, or consisting essentially of, an amino acid sequence as set forth in **SEQ ID NO: 8** (GKPIPNPLLGLDST), a fragment or a variant thereof,
- a T2A self-cleaving peptide amino acid sequence comprising, or consisting essentially of, an amino acid sequence as set forth in **SEQ ID NO: 9** (EGRGSLLTCGDVEENPGP), a fragment or a variant thereof, and
- a SV40 NLS amino acid sequence comprising, or consisting essentially of, an amino acid sequence as set forth in **SEQ ID NO: 10** (PKKKRKV) a fragment or a variant thereof.

Non limiting examples of a fusion protein of the invention are selected from the group comprising
i) a dCas9-KRAB-SCAN fusion protein as set forth in **SEQ ID NO: 4** or an amino acid sequence having at least 95%, 96%, 97%, 98%, 99%, or 100% sequence identity thereto;
ii) a dCas9 -SCAN fusion protein as set forth in **SEQ ID NO: 5** or an amino acid sequence having at least 95%, 96%, 97%, 98%, 99%, or 100% sequence identity thereto; or iii) a DamID-SCAN fusion protein as set forth in **SEQ ID NO: 6** or an amino acid sequence having at least 95%, 96%, 97%, 98%, 99%, or 100% sequence identity thereto.

The present invention also contemplates one or more polynucleotides encoding a fusion protein according to the invention as well as a fragment or a variant of said one or more polynucleotides.

The present invention further provides a plasmid or a vector comprising a nucleic acid sequence encoding at least one fusion protein according to the invention.

Any vector or plasmid known in the art can be suitable for the present invention. In one aspect, the vector is selected from the group comprising a retroviral vector (such as pMSGV), a DNA vector, a murine leukemia virus vector, an SFG vector, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector (such as pGAR), or any combination thereof.

The present invention further provides a prokaryotic or eukaryotic host cell, or population of cells, comprising the plasmid or the vector of the invention. In one aspect, the host cell or population of cells comprises a mammalian cell, an amphibian cell, a fish cell, an insect cell, a plant cell, or a yeast cell.

Insertion of a vector or plasmid is usually called transformation for prokaryotic cells and transfection for eukaryotic cells, although insertion of a viral vector may also be called transduction. The skilled person will be aware of the different non-viral transfection methods commonly used, which include, but are not limited to, the use of physical methods (e.g., electroporation, cell squeezing, sonoporation, optical transfection, protoplast fusion, impalefection, magnetofection, gene gun or particle bombardment), chemical reagents (e.g., calcium phosphate, highly branched organic compounds or cationic polymers) or cationic lipids (e.g., lipofection). Many transfection methods require the contact of solutions of plasmid DNA or vectors to the cells, which are then grown and selected for a marker gene expression.

The present invention further provides a composition comprising a fusion protein according to the invention and at least one or more gRNAs that bind the catalytically inactive endonuclease, a polynucleotide of the invention, a plasmid or vector of the invention, or a prokaryotic or eukaryotic host cell, or population of cells, as disclosed herein.

The present invention also provides pharmaceutical composition comprising a therapeutically effective amount of i) a fusion protein according to the invention and at least one or more gRNAs that bind the catalytically inactive endonuclease, ii) a polynucleotide of the invention, iii) a plasmid or vector of the invention, or iv) a prokaryotic or eukaryotic host cell, or population of cells, as disclosed herein, and a pharmaceutically acceptable carrier, diluent and/or excipient.

According to an aspect, the pharmaceutical composition is for use in the treatment and / or prevention of cancer, neurodegenerative disease, neurodevelopmental disease, aging and aging-related disease.

As used herein, a guide RNA (gRNA), or single guide RNA (sgRNA), is any polynucleotide sequence having sufficient complementarity with a target polynucleotide sequence to hybridize with the target sequence and direct sequence-specific binding of a CRISPR complex to the target sequence. Typically, the gRNA is a single chimeric RNA formed by the fusion of tracrRNA and crRNA containing two major regions of importance for CRISPR systems, the scaffold and spacer regions, that are used to direct dCas9 to their target sites. The spacer region contains nucleotides that are complimentary to those found in the target sites of the gene(s) of interest. The scaffold region is responsible for the formation of a complex with dCas9. Together, they bind dCas9 and direct it to the gene(s) of interest.

The sequence within the sgRNA that is responsible for Cas9 binding is similarly responsible for dCas9 complex formation. The CRISPR/Cas9 systems possess high flexibility compared to other genome engineering tools because any genes or nucleotides with a sequence complimentary to the spacer region can become possible targets for gene editing through modification of the sgRNA spacer region for any potential sequence.

In some aspects, to generate gRNAs that target specific genes of interest, one or more gRNA libraries containing targeting sequences can be screened according to the protocols described in Replogle et al. Nature Biotechnology.2020 (see also Sanger Arrayed Whole Genome Lentiviral CRISPR Library by Sigma). In one aspect, a specific gRNA is selected from gRNA libraries. Usually, the sgRNA recognizes a target sequence composed of 16 to 25 nucleotides (e.g. 16, 17, 18, 19, 20, 21 , 22, 23, 24 or 25 nucleotides), depending on which species the Cas9 comes from.

The terms "recognition sequence", "recognition site", "nuclease target site", or "target site" are used herein to refer to a location within a nucleic acid sequence where a nuclease interacts with a specific nucleotide sequence.

In the context of the present application, a gene of interest means any gene associated with a disease, whether monogenic or multigenic.

The present invention further contemplates methods of treatment and/or prevention of a disease comprising administering toa subject in need thereof, a i) pharmaceutical composition of the invention, ii) a fusion protein along with at least one or more gRNAs of the invention, iii) a polynucleotide of the invention, iv) a plasmid or vector of the invention, or v) a prokaryotic or eukaryotic host cell, or population of cells.

The term "treatment" or "treating" means any administration of a composition, pharmaceutical composition, fusion protein along with at least one or more gRNAs, a polynucleotide of the invention, a plasmid or vector of the invention, or a prokaryotic or eukaryotic host cell, or population of cells, *etc...* of the disclosure to a subject for the purpose of:
(i) inhibiting the disease, that is, arresting the development of clinical symptoms; and/or
(ii) relieving the disease, that is, causing the regression of clinical symptoms.

As used herein, the term "prevention" or "preventing" means any administration of a composition, a pharmaceutical composition, a fusion protein along with at least one or more gRNAs, a polynucleotide of the invention, a plasmid or vector of the invention, or a prokaryotic or eukaryotic host cell, or population of cells, *etc...* of the disclosure to a subject for the purpose of preventing the disease, that is, causing the clinical symptoms of the disease not to develop.

In the context of the present invention, the disease can be any disease, whether monogenic or multigenic. In one aspect, the disease is cancer, a neurodegenerative disease, a neurodevelopmental disease, or an aging-related disease.

In another aspect, the fusion protein of the invention comprises a SCAN domain bearing member of a zinc finger-containing protein, a fragment or a variant thereof and a DNA adenine methyltransferase, a fragment or a variant thereof.

DamID-SCAN fusion protein is constructed consisting of *E. coli* DNA adenine methyltransferase (dam) and the SCAN domain of ZNF274. Expression of this fusion protein *in vivo* leads to preferential methylation of adenines in DNA surrounding nucleoli due to the ability of SCAN to localize the fusion partner in this subnuclear compartment. DamID-SCAN interacts with DNA independently of the sequence content. Because adenine methylation does not occur endogenously in most eukaryotes, it provides a unique tag to mark protein interaction sites. The adenine methylated DNA fragments can be isolated and identified by high-throughput sequencing methods to map genome-wide interaction sites. The application of DamID-SCAN is relevant to identify NADs in many cell types, including single-cell measurements which remain prohibitive with biochemical based-methods.

The present invention further provides an expression cassette.

As used herein, an "expression cassette" refers to a nucleic acid molecule which encodes a SCAN domain bearing member of a zinc finger-containing protein, a fragment or variant thereof and ii) a catalytically inactive RNA-guided endonuclease or iii) a DNA adenine methyltransferase.

An expression cassette also contains a promoter and may contain additional regulatory elements that control expression of the gene editing system in a host cell. In one aspect, the expression cassette may be packaged into the capsid of a viral vector (e.g., a viral particle). In one aspect, such an expression cassette for generating a viral vector as described herein is flanked by packaging signals of the viral genome and other expression control sequences such as those described herein. For example, for an AAV viral vector, the packaging signals are a 5' AAV inverted terminal repeat (ITR) and a 3' AAV ITR.

An expression cassette can further contain a sequence encoding an endonuclease (e.g. dCas9) and targeting sequence (e.g. crRNA sequence of a CRISPR/Cas system, a Cas9 nuclease-recruiting sequence (tracrRNA), a sgRNA containing both a crRNA sequence and a tracrRNA sequence, ...).

Non-limiting examples of expression cassettes comprise a nucleic acid molecule which encodes i) a dCas9-KRAB-SCAN fusion protein as set forth in **SEQ ID NO: 4** or an amino acid sequence having at least 95%, 96%, 97%, 98%, 99%, or 100% sequence identity thereto; ii) a dCas9 -SCAN fusion protein as set forth in **SEQ ID NO: 5** or an amino acid sequence having at least 95%, 96%, 97%, 98%, 99%, or 100% sequence identity thereto; or iii) a DamID-SCAN fusion protein as set forth in **SEQ ID NO: 6** or an amino acid sequence having at least 95%, 96%, 97%, 98%, 99%, or 100% sequence identity thereto.

The expression cassette further contains a promoter and may contain additional regulatory elements known in the art and/or an endonuclease and targeting sequence described herein.

The present invention also provides a method of repressing the expression of a gene in a cell, comprising contacting the cell with an effective amount of:
(a) the fusion protein of the invention or the polynucleotide encoding said fusion protein; and
(b) one or more gRNAs that bind the dCas9 protein according to the invention.

In one aspect, the gene is a gene associated with a disease.
dCas9 is a powerful tool not only for transcriptional activation but also for targeted repression of gene transcription. This inhibition occurs when binding of dCas9 to the target site sterically interferes with the binding and function of the transcriptional machinery; dCas9 alone functions as a repressor and blocks transcription through CRISPR interference. CRISPRi has been reported to be used for effective, stable RNA-guided transcriptional suppression of a target gene. The suppression of transcription is inducible and reversible, and the recognition of targets only depends on the sgRNA sequence. Recruitment of dCas9 to the recognition complex containing sgRNA inhibits gene expression by interfering with transcriptional elongation, RNA polymerase binding, or transcription factor binding (Khatodia S, Bhatotia K, Passricha N, Khurana SM, Tuteja N. The CRISPR/Cas Genome-Editing Tool: Application in Improvement of Crops. Front Plant Sci. 2016 Apr 19;7:506.; Laganà A, Shasha D, Croce CM. Synthetic RNAs for Gene Regulation: Design Principles and Computational Tools. Front Bioeng Biotechnol. 2014 Dec 11 ; ; Qi LS, Larson MH, Gilbert LA, Doudna JA, Weissman JS, Arkin AP, Lim WA. Repurposing CRISPR as an RNA-guided platform for sequence-specific control of gene expression. Cell. 2013 Feb 28;152(5):1173-83).

The invention also contemplates kits for performing a method according to the invention or for the treatment and/or prevention of a disease of the invention. In one aspect of the invention, the kit comprises a pharmaceutical composition comprising a therapeutically effective amount of at least one fusion protein and at least one or more gRNAs that bind the catalytically inactive endonuclease as described herein, a polynucleotide as described herein, a plasmid or vector as described herein, or a prokaryotic or eukaryotic host cell, or population of cells, as described herein.

The kits of the invention may also comprise a container and a label or package insert on or associated with the container. Suitable containers include, for example, bottles, vials, syringes, etc. The containers may be formed from a variety of materials such as glass or plastic. The container holds a composition which is effective for treating the disease of disorder of the invention and may have a sterile access port (for example the container may be an intravenous solution bag or a vial having a stopper pierceable by a hypodermic injection needle). Alternatively, or additionally, the kits may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The label or package insert may comprise instructions for use thereof. Instructions included may be affixed to packaging material or may be included as a package insert. While the instructions are typically written or printed materials they are not limited to such. Any medium capable of storing such instructions and communicating them to an end user is contemplated by this disclosure.

The invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification, each of which is incorporated herein by reference in its entirety. The foregoing description will be more fully understood with reference to the following Examples.

### EXAMPLES

### Material & Methods

### Plasmid Construction of CRISPR-S constructs

The human codon-optimized sequence of the SCAN domain from ZNF274 is used either to be fused with the sequence-encoding human codon-optimized Streptococcus pyogenes dCas9-KRAB (pLV hU6-sgRNA hUbC-dCas9-KRAB-T2a-Puro from Addgene, plasmid #71236) or to replace the KRAB domain using In-Fusion cloning (Clontech).
- Amplify SCAN using pTRE-ZNF274-3HA plasmid (from Imbeault et al., Nature 2017) as template DNA.
- The following primers are used for cloning the dCas9-SCAN construct:
   ∘ INF_dCas9-274SCAN_F:
      5'-GAGGAAGGTGGctagcAGACAGCGGTTCCGGC-3' (**SEQ ID NO: 11**)
   ∘ INF_dCas9-274SCAN_R:
      5'-CTCTGCCCTCGctagCGCTCATCCATGTCACGC-3' (**SEQ ID NO: 12**)
- The following primers are used for cloning the dCas9-KRAB-SCAN construct:
   ∘ INF_dCAs9KRAB-274SCAN F1:
      5'-GAGGAAGGTGGctagCGATGCTAAGTCACTG-3' **(SEQ ID NO: 13)**
   ∘ INF_dCAs9KRAB-274SCAN R1:
      5'-CGCTGTCTaaCTTTGCGTTTCTTTTTCGGAACTGAT-3'**(SEQ ID NO: 14)**
   ∘ INF_dCAs9KRAB-274SCAN F2:
      5'-CAAAGttAGACAGCGGTTCCGGC-3'**(SEQ ID NO: 15)**
   ∘ INF_dCAs9KRAB-274SCAN R2:
      5'-CTCTGCCCTCGctagCGCTCATCCATGTCACGC-3'(SEQ ID NO: 16)

### CRISPR-S silencing of POU5F1B gene in LS1034 cell line

sgRNAs are cloned into a lentiviral U6-based expression vector (pLenti-SpBsmBI-sgRNA-Hygro vector from Addgene, plasmid #62205).

One sgRNA was used to target POU5F1B gene:
- LTR-g1: 5'- TCACATCATTCTCACCACTCTGG-3' (SEQ ID NO: 17)

LS1034 cells are maintained in RPMI 1640 medium (Gibco) supplemented with 10% FCS (Bioconcept 2-01F36-I).

Lentiviral vectors produced from pLV-hU6-sgRNA-hUbC-dCas9-KRAB-SCAN or pLV-hU6-sgRNA-hUbC-dCas9-SCAN are used to transduce LS1034 cells. After 72 hours, transduced cells are selected in 1 µg/ml puromcycin for 7 days before transfection with sgRNA-producing plasmids. After 24 hours, transfected cells are selected in 200 µg/ml hygromycin for 7 days.

Expression levels of POU5F1B are checked by quantitative real-time PCR using primers P1
(5'-GGCCTCACACCGAATAACTC-3'**(SEQ ID NO: 18**), 5'-ACGAGGAGCAGTCTCCTGAA-3' **(SEQ ID NO: 19**))
and P2 (5'-GAGCCAAGAGAAGACGTCCAG-3' **(SEQ ID NO: 20)**, 5'-GCTTGTGACTTAGCCTGGGTG-3'**(SEQ ID NO: 21)**)**.**

### Plasmid Construction of DAMID-SCAN construct

SCAN was amplified using pTRE-ZNF274-3HA plasmid (from Imbeault et al., Nature 2017) as template DNA using the following primers:
∘ Attb_FOR: 5'- CACCAGACAGCGGTTCCGG-3'**(SEQ ID NO: 22)**
∘ Attb_REV: 5'- GCTCATCCATGTCACGCCTTCC-3'**(SEQ ID NO: 23)**. Clone SCAN PCR fragment into the pENTR/D-TOPO vector using BP Clonase II.

Clone SCAN from the donor vector pENTR-SCAN into the pLGW-RFC1-VS-EcoDam (Addgene plasmid #59205) destination vector or the pLGW-EcoDam-VS-RFC1 (Addgene plasmid #59209) destination vectors LR Clonase II.

### Results

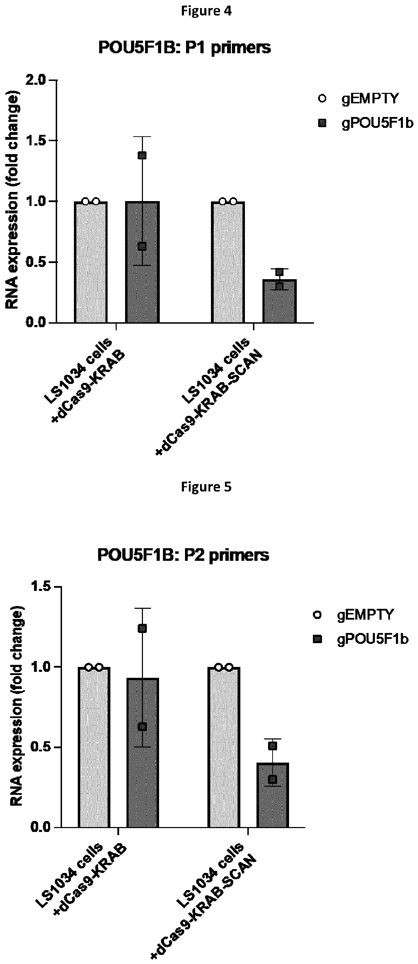

Expression of POU5F1b RNA is decreased in LS1034 cells when dCas9-KRAB-SCAN is targeted to its LTR66 promoter compared to dCas9-KRAB using the same sgRNA (n = 2 independent experiments). The impact of indicated manipulations on POU5F1b RNA levels is checked by quantitative real-time PCR using primers P1 and P2 (Figs. 4 & 5).

### Sequences

- Amino acid sequence of ZNF274 (**SEQ ID NO: 24**): 
MASRLPTAWSCEPVTFEDVTLGFTPEEWGLLDLKQKSLYREVMLENYRNLVSVEHQ
LSKPDVVSQLEEAEDFWPVERGIPQDTIPEYPELQLDPKLDPLPAESPLMNIEVVEVLT
LNQEVAGPRNAQIQALYAEDGSLSADAPSEQVQQQGKHPGDPEAARQRFRQFRYKD
MTGPREALDQLRELCHQWLQPKARSKEQILELLVLEQFLGALPVKLRTWVESQHPE
NCQEVVALVEGVTWMSEEEVLPAGQPAEGTTCCLEVTAQQEEKQEDAAICPVTVLP
EEPVTFQDVAVDFSREEWGLLGPTQRTEYRDVMLETFGHLVSVGWETTLENKELAP
NSDIPEEEPAPSLKVQESSRDCALSSTLEDTLQGGVQEVQDTVLKQMESAQEKDLPQ
KKHFDNRESQANSGALDTNQVSLQKIDNPESQANSGALDTNQVLLHKIPPRKRLRKR
DSQVKSMKHNSRVKIHQKSCERQKAKEGNGCRKTFSRSTKQITFIRIHKGSQVCRCS
ECGKIFRNPRYFSVHKKIHTGERPYVCQDCGKGFVQSSSLTQHQRVHSGERPFECQE
CGRTFNDRSAISQHLRTHTGAKPYKCQDCGKAFRQSSHLIRHQRTHTGERPYACNKC
GKAFTQSSHLIGHQRTHNRTKRKKKQPTS
- Amino acid sequence of the SCAN domain of ZNF274 (SEQ ID NO: 1):
   RQRFRQFRYKDMTGPREALDQLRELCHQWLQPKARSKEQILELLVLEQFLGALPVK LRTWVESQHPENCQEVVALVEGVTWMS
- Amino acid sequence of dCas9-SCAN-T2A-PURO (SEQ ID NO: 25): 
DYKDHDGDYKDHDIDYKDDDDKMAPKKKRKVGRGMDKKYSIGLAIGTNSVGWAV
ITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIY
HLRKKLVDSTDKADLRLIYLALAHMIKFRGHFLIEGDLNPDNSDVDKLFIQLVQTYN
QLFEENPINASGVDAKAILSARLSKSRRLENLIAQLPGEKKNGLFGNLIALSLGLTPNF
KSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRR
QEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKA
 
QVSGQGDSLHEHIANLAGSPAIKKGILQTVKVVDELVKVMGRHKPENIVIEMARENQ
TTQKGQKNSRERMKRIEEGIKELGSQILKEHPVENTQLQNEKLYLYYLQNGRDMYV
DQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDS
RMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKE
SILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQK
GNELALPSKYVNFLYLASHYEKLKGSPEDNEQKQLFVEQHKHYLDEIIEQISEFSKRV
ILADANLDKVLSAYNKHRDKPIREQAENIIHLFTLTNLGAPAAFKYFDTTIDRKRYTS
TKEVLDATLIHQSITGLYETRIDLSQLGGDSRADPKKKRKVASRQRFRQFRYKDMTG
PRELDQLRELCHQWLQPKARSKEQILELLVLEQFLGALPVKLRTWVESQHPENCQEV
VALVEGVTWMSASEGRGSLLTCGDVEENPGPMTEYKPTVRLATRDDVPRAVRTLA
AAFADYPATRHTVDPDRHIERVTELQELFLTRVGLDIGKVWVADDGAAVAVWTTPE
SVEAGAVFAEIGPRMAELSGSRLAAQQQMEGLLAPHRPKEPAWFLATVGVSPDHQG
KGLGSAVVLPGVEAAERAGVPAFLETSAPRNLPFYERLGFTVTADVEVPEGPRTWC
MTRKPGA
- Amino acid sequence of dCas9-SCAN (**SEQ ID NO: 5**): 
DYKDHDGDYKDHDIDYKDDDDKMAPKKKRKVGRGMDKKYSIGLAIGTNSVGWAV
ITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIY
HLRKKLVDSTDKADLRLIYLALAHMIKFRGHFLIEGDLNPDNSDVDKLFIQLVQTYN
QLFEENPINASGVDAKAILSARLSKSRRLENLIAQLPGEKKNGLFGNLIALSLGLTPNF
KSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRR
QEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQ
 
TTQKGQKNSRERMKRIEEGIKELGSQILKEHPVENTQLQNEKLYLYYLQNGRDMYV
DQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDS
RMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKE
SILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQK
GNELALPSKYVNFLYLASHYEKLKGSPEDNEQKQLFVEQHKHYLDEIIEQISEFSKRV
ILADANLDKVLSAYNKHRDKPIREQAENIIHLFTLTNLGAPAAFKYFDTTIDRKRYTS
TKEVLDATLIHQSITGLYETRIDLSQLGGDSRADPKKKRKVASRQRFRQFRYKDMTG
PRELDQLRELCHQWLQPKARSKEQILELLVLEQFLGALPVKLRTWVESQHPENCQEV
VALVEGVTWMS
- Amino acid sequence of dCas9-KRAB-SCAN-T2A-PURO (**SEQ ID NO: 26**): 
DYKDHDGDYKDHDIDYKDDDDKMAPKKKRKVGRGMDKKYSIGLAIGTNSVGWAV
ITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIY
HLRKKLVDSTDKADLRLIYLALAHMIKFRGHFLIEGDLNPDNSDVDKLFIQLVQTYN
QLFEENPINASGVDAKAILSARLSKSRRLENLIAQLPGEKKNGLFGNLIALSLGLTPNF
KSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRR
QEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQ
TTQKGQKNSRERMKRIEEGIKELGSQILKEHPVENTQLQNEKLYLYYLQNGRDMYV
DQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDS
RMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEIT
 
LANGEIRKRPLIETNGETGEIVWDKGRDFATVRKVLSMPQVNIVKKTEVQTGGFSKE
SILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQK
GNELALPSKYVNFLYLASHYEKLKGSPEDNEQKQLFVEQHKHYLDEIIEQISEFSKRV
ILADANLDKVLSAYNKHRDKPIREQAENIIHLFTLTNLGAPAAFKYFDTTIDRKRYTS
TKEVLDATLIHQSITGLYETRIDLSQLGGDSRADPKKKRKVASDAKSLTAWSRTLVT
FKDVFVDFTREEWKLLDTAQQILYRNVMLENYKNLVSLGYQLTKPDVILRLEKGEE
PWLVEREIHQETHPDSETAFEIKSSVPKKKRKVRQRFRQFRYKDMTGPRELDQLREL
CHQWLQPKARSKEQILELLVLEQFLGALPVKLRTWVESQHPENCQEVVALVEGVTW
MSASEGRGSLLTCGDVEENPGPMTEYKPTVRLATRDDVPRAVRTLAAAFADYPATR
HTVDPDRHIERVTELQELFLTRVGLDIGKVWVADDGAAVAVWTTPESVEAGAVFAE
IGPRMAELSGSRLAAQQQMEGLLAPHRPKEPAWFLATVGVSPDHQGKGLGSAVVLP
GVEAAERAGVPAFLETSAPRNLPFYERLGFTVTADVEVPEGPRTWCMTRKPGA
- Amino acid sequence of dCas9-KRAB-SCAN (SEQ ID NO: 4): 
DYKDHDGDYKDHDIDYKDDDDKMAPKKKRKVGRGMDKKYSIGLAIGTNSVGWAV
ITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIY
HLRKKLVDSTDKADLRLIYLALAHMIKFRGHFLIEGDLNPDNSDVDKLFIQLVQTYN
QLFEENPINASGVDAKAILSARLSKSRRLENLIAQLPGEKKNGLFGNLIALSLGLTPNF
KSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRR
QEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQ
TTQKGQKNSRERMKRIEEGIKELGSQILKEHPVENTQLQNEKLYLYYLQNGRDMYV
DQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDS
RMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEIT
 
LANGEIRKRPLIETNGETGEIVWDKGRDFATVRKVLSMPQVNIVKKTEVQTGGFSKE
SILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQK
GNELALPSKYVNFLYLASHYEKLKGSPEDNEQKQLFVEQHKHYLDEIIEQISEFSKRV
ILADANLDKVLSAYNKHRDKPIREQAENIIHLFTLTNLGAPAAFKYFDTTIDRKRYTS
TKEVLDATLIHQSITGLYETRIDLSQLGGDSRADPKKKRKVASDAKSLTAWSRTLVT
FKDVFVDFTREEWKLLDTAQQILYRNVMLENYKNLVSLGYQLTKPDVILRLEKGEE
PWLVEREIHQETHPDSETAFEIKSSVPKKKRKVRQRFRQFRYKDMTGPRELDQLREL
CHQWLQPKARSKEQILELLVLEQFLGALPVKLRTWVESQHPENCQEVVALVEGVTW
MS
- Amino acid sequence of DamID-SCAN (**SEQ ID NO: 6**): 
MKKNRAFLKWAGGKYPLLDDIKRHLPKGECLVEPFVGAGSVFLNTDFSRYILADINS
DLISLYNIVKMRTDEYVQAARELFVPETNCAEVYYQFREEFNKSQDPFRRAVLFLYL
NRYGYNGLCRYNLRGEFNVPFGRYKKPYFPEAELYHFAEKAQNAFFYCESYADSMA
RADDASVVYCDPPYAPLSATANFTAYHTNSFTLEQQAHLAEIAEGLVERHIPVLISNH
DTMLTREWYQRAKLHVVKVRRSISSNGGTRKKVDELLALYKPGVVSPAKKTGGKPI
PNPLLGLDSTIDFDHPSLYKKAGSAAAPFTRQRFRQFRYKDMTGPRELDQLRELCHQ
WLQPKARSKEQILELLVLEQFLGALPVKLRTWVESQHPENCQEVVALVEGVTWMSK
GGRADPAFLYKVVRSNRGLDSRPRKTWSNHK
- Amino acid sequence of dCas9 (**SEQ ID NO: 3**):

MDKKYSIGLAIGTNSVGWAVITDEYKVPSKKFKVLGNTDRHSIKKNLIGALLFDSGE
TAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQ
RTFDNGSIPHQIHLGELHAILRRQEDFYPFLKDNREKIEKILTFRIPYYVGPLARGNSRF
AWMTRKSEETITPWNFEEVVDKGASAQSFIERMTNFDKNLPNEKVLPKHSLLYEYFT
VYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEE
RLKTYAHLFDDKVMKQLKRRRYTGWGRLSRKLINGIRDKQSGKTILDFLKSDGFAN

RNFMQLIHDDSLTFKEDIQKAQVSGQGDSLHEHIANLAGSPAIKKGILQTVKVVDEL
VKVMGRHKPENIVIEMARENQTTQKGQKNSRERMKRIEEGIKELGSQILKEHPVENT
QLQNEKLYLYYLQNGRDMYVDQELDINRLSDYDVDAIVPQSFLKDDSIDNKVLTRS
DKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSEQEI
GKATAKYFFYSNIMNFFKTEITLANGEIRKRPLIETNGETGEIVWDKGRDFATVRKVL
SMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYS
LFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLTN
LGAPAAFKYFDTTIDRKRYTSTKEVLDATLIHQSITGLYETRIDLSQLGGDSRAD

## Claims

1. A fusion protein comprising
i) a SCAN domain bearing member of a zinc finger-containing protein (ZFP), a fragment or variant thereof and
ii) a catalytically inactive RNA-guided endonuclease or
iii) a DNA adenine methyltransferase.

2. The fusion protein according to claim 1, wherein the catalytically inactive endonuclease is a Cas9 protein or a variant thereof.

3. The fusion protein according to claim 1 or 2, wherein the SCAN domain is able to drive nucleolar localization and/or protein dimerization of a transcription factor.

4. The fusion protein according to any one of claims 1 to 3, wherein the SCAN domain is selected from ZNF274 or the group comprising any other SCAN-containing proteins capable of interacting with the SCAN domain of ZNF274 or whose perinucleolar tethering depends on ZNF274.

5. The fusion protein according to claim 4, wherein the SCAN domain of ZNF274 comprises, or essentially consists of, an amino acid sequence as set forth in **SEQ ID NO: 1**, a fragment or a variant thereof.

6. The fusion protein according to any one of claims 1 to 5, wherein the DNA adenine methyltransferase is selected from the group comprising the DNA adenine methyltransferase identification (DamID), a fragment or a variant thereof.

7. The fusion protein according to claim 6, wherein the DNA adenine methyltransferase identification (DamID) domain comprises, or essentially consists of, an amino acid sequence as set forth in **SEQ ID NO: 2**, a fragment or a variant thereof.

8. The fusion protein according to any one of claims 1 to 7, further comprising a repressor domain selected from the group comprising a Krüppel-associated box (KRAB) domain, a transcription repression domain of methyl-CpG binding protein 2, or regulatory domains of other proteins (e.g. a transcriptional repression domain) or a combination of one or more thereof.

9. The fusion protein according to any one of claims 1 to 8, wherein the Cas9 protein comprises at least one domain selected from the group comprising a Rec1 domain, a bridge helix domain, and/or a protospacer adjacent motif (PAM) interacting domain.

10. The fusion protein according to any one of claims 1 to 9, wherein the Cas9 is a dCas9 protein comprising a D10A mutation in a RuvC1 domain and/or a H840A mutation in a HNH domain.

11. The fusion protein according to claim 9 or 10, wherein the dCas9 protein comprises, or essentially consists of, an amino acid sequence as set forth in SEQ ID NO: 3, a fragment or a variant thereof.

12. The fusion protein according to any one of claims 1 to 11, further comprising one or more linkers.

13. The fusion protein according to any one of claims 1 to 12, wherein the fusion protein is selected from the group comprising
i) a dCas9-KRAB-SCAN fusion protein as set forth in **SEQ ID NO. 4** or an amino acid fragment sequence having at least 80%, 8195%, 96%, 97%, 98%, 99%, or 100% sequence identity thereto;
ii) a dCas9 -SCAN fusion protein as set forth in **SEQ ID NO. 5** or an amino acid fragment sequence having at least 95%, 96%, 97%, 98%, 99%, or 100% sequence identity thereto; or
iii) a DamID-SCAN fusion protein as set forth in **SEQ ID NO. 6** or an amino acid fragment sequence having at least 95%, 96%, 97%, 98%, 99%, or 100% sequence identity thereto.

14. An isolated polynucleotide encoding a fusion protein according to any one of claims 1 to 13.

15. A plasmid or a vector comprising a nucleic acid sequence of claim 14.

16. A prokaryotic or eukaryotic host cell, or population of cells, comprising the plasmid or vector of claim 15.

17. A pharmaceutical composition comprising a therapeutically effective amount of at least one fusion protein according to any one of claims 1 to 13 and at least one or more gRNAs that bind the catalytically inactive endonuclease, a polynucleotide of claim 14, a plasmid or vector of claim 15, or a prokaryotic or eukaryotic host cell, or population of cells, of claim 16, and a pharmaceutically acceptable carrier, diluent and/or excipient.

18. The pharmaceutical composition according to claim 17 for use in the treatment and / or prevention of cancer, neurodegenerative disease, neurodevelopmental disease, aging and aging-related disease.
